# FASCICULE DE BREVET EUROPEEN

(11) **EP 2 380 608 B1**
(45) Date de publication et mention de la délivrance du brevet: **12.09.2012**
(21) Numéro de dépôt: 11161291.7
(22) Date de dépôt: 06.04.2011
(51) Int. Cl.: A61L 15/40, A61L 27/36, A61L 27/18

(54) **Procédé pour l'obtention d'un materiau hémocompatible composite et matériau obtenu.**
Hemokompatibles-Composite-Material sowie Verfahren zu dessen Herstellung
Process to form an hemocompatible composite material and material obtained.

(30) Priorité: 22.04.2010 FR 1001724
(43) Date de publication de la demande: 26.10.2011
(73) Titulaire: CARMAT, 78941 Vélizy Villacoublay cedex (FR)
(72) Inventeur: MELOT, Marion, 92130 ISSY LES MOULINEAUX (FR); CAPEL, Antoine, 33600 PESSAC (FR)
(74) Mandataire: Gevers France

(56) Documents cités:
- EP-A1- 1 785 154
- WO-A2-2004/003178

## Description

La présente invention concerne un procédé pour l'obtention d'un matériau hémocompatible composite, ainsi qu'un tel matériau obtenu par la mise en oeuvre dudit procédé.

On sait que le sang est un tissu liquide vivant très sensible et qu'il est aisément altéré au contact de substances chimiques ou lors d'une exposition à des contraintes mécaniques, par exemple de cisaillement ; il coagule au contact de la plupart des matériaux inertes ou lors de stases. En réalité, il existe très peu de matériaux hémocompatibles et la plupart d'entre eux nécessitent la prise d'anticoagulants par un patient porteur d'un tel matériau hémocompatible.

On sait de plus (voir par exemple le document US-A-5 135 539) qu'il existe des prothèses cardiaques dans lesquelles les ventricules artificiels comportent des membranes souples de matériau hémocompatible, actionnées par des impulsions d'un fluide pour mettre le sang en mouvement. Dans ce cas, l'hémocompatibilité desdites membranes est particulièrement critique, du fait que les membranes sont mobiles et au contact d'un flux sanguin complexe et souvent turbulent.

Dans la technique, on connaît des matériaux sensiblement hémocompatibles qui sont soit synthétiques, soit d'origine biologique.

Les matériaux synthétiques sont généralement des élastomères de polyuréthane ou de silicone ; ils sont utilisés soit avec une surface lisse, afin de réduire les adhésions plaquettaires ou sanguines, soit avec une surface poreuse, afin de permettre l'adhésion d'une couche biologique apte à servir d'interface avec le sang. De tels matériaux synthétiques présentent de bonnes qualités de souplesse, d'imperméabilité et de déformabilité, mais nécessitent l'utilisation d'anticoagulants.

Les matériaux d'origine biologique sont des tissus d'animaux ou sont recomposés à partir de matière biologique, par exemple le collagène. Les tissus de nature animale doivent être fixés chimiquement (le plus souvent à l'aide de glutaraldéhyde) lorsqu'ils ont pour but d'être implantés dans le corps humain, afin d'éviter les réactions immunologiques. De tels matériaux biologiques ainsi traités présentent généralement d'excellentes propriétés hémocompatibles et ne nécessitent d'ailleurs pas l'utilisation d'anticoagulants par le patient, mais ils ne sont absolument pas imperméables.

A contrario, les matériaux synthétiques, dits hémocompatibles et implantables, ont généralement des caractéristiques mécaniques et d'étanchéité intéressantes, mais ne sont tolérés dans le flux sanguin qu'au moyen d'une anticoagulation rigoureuse.

Pour pouvoir bénéficier des bonnes propriétés mécaniques et d'étanchéité des matériaux synthétiques et des bonnes caractéristiques d'hémocompatibilité des matériaux d'origine biologique, le document US-A-5 135 539 prévoit de superposer une membrane de matériau synthétique et une membrane d'origine biologique. Cependant, une telle disposition entraîne la formation d'une chambre intermédiaire entre lesdites membranes, qui peut être le siège d'infections ou de collections liquidiennes indésirables.

Pour remédier à tous les inconvénients de la technique antérieure précitée, le brevet européen EP 1 785 154 décrit un matériau hémocompatible comportant un substrat synthétique résistant, souple et étanche, par exemple réalisé en un élastomère de polyuréthane ou de silicone, et un tissu biologique animal, par exemple du péricarde animal, ledit tissu biologique étant rendu solidaire dudit substrat par une dispersion de la matière constitutive de ce dernier dans un solvant, ladite matière constitutive imprégnant ledit tissu biologique animal.

Ainsi, grâce au document EP 1 785 154, on obtient un matériau composite dont l'hémocompatibilité est assurée par le tissu biologique, alors que la résistance mécanique et l'étanchéité sont apportées par le substrat synthétique. On remarquera de plus que, lorsque ledit tissu biologique est constitué de péricarde animal, par exemple de péricarde bovin, ce tissu biologique est lui-même résistant et participe à la résistance mécanique dudit matériau composite.

Pour permettre la solidarisation dudit tissu biologique animal sur ledit substrat synthétique au moyen de ladite dispersion, il est indispensable de déshydrater ledit tissu biologique animal. Pour ce faire, le document EP 1 785 154 prévoit de lyophiliser ledit tissu biologique animal, ce qui non seulement déshydrate celui-ci, mais encore permet de conserver la structure tridimensionnelle dudit tissu biologique après déshydratation. En effet, lorsqu'un tissu biologique se déshydrate dans des conditions ordinaires, les fibres de collagène qui le constituent viennent au contact les unes des autres et il se crée des liaisons chimiques irréversibles rendant impossible la réhydratation ultérieure du tissu biologique. La lyophilisation, au contraire, permet d'immobiliser la structure du tissu biologique animal par congélation, puis de retirer l'eau à très basse pression par sublimation, donc sans permettre de mobilité ni de réarrangement des fibres. Toutefois, la durée de l'étape de lyophilisation est longue (au moins 96 heures) et il est nécessaire de mettre en oeuvre une infrastructure spécifique et coûteuse. De plus, la conduite de la lyophilisation est délicate, car la déshydratation ne doit pas être complète sous peine d'endommager irrémédiablement le tissu biologique animal. Or, l'efficacité de la déshydratation dépend de l'épaisseur et de la nature dudit tissu biologique, de sorte qu'elle est difficile à maîtriser et que l'étape de lyophilisation s'accompagne inévitablement d'un pourcentage relativement élevé de rebuts. De plus, une telle déshydratation non complète du tissu biologique animal rend celui-ci instable, de sorte que son stockage et son transport à l'état lyophilisé sont complexes et nécessitent une mise sous vide.

La présente invention a pour objet de remédier à ces inconvénients.

A cette fin, selon l'invention, le procédé pour la réalisation d'un matériau hémocompatible comportant un substrat synthétique, résistant et étanche, et un tissu biologique animal, fixé chimiquement afin d'éviter des réactions immunologiques, procédé selon lequel on déshydrate ledit tissu animal, on colle ledit tissu biologique animal déshydraté sur ledit substrat synthétique au moyen d'une dispersion de la matière constitutive dudit substrat synthétique dans un solvant de manière que ladite matière constitutive imprègne ledit tissu biologique animal, puis on élimine ledit solvant, est remarquable en ce que la déshydratation est obtenue uniquement par voie chimique par immersion dudit tissu biologique animal dans un bain constitué d'une solution de polyéthylène glycol à au moins 80% en poids.

Ainsi, ladite solution de polyéthylène glycol à au moins 80% en poids permet d'obtenir rapidement (de l'ordre de 24 heures) une membrane de tissu biologique animal, qui ne contient pas d'eau, ce qui est indispensable pour le collage sur le substrat synthétique, mais qui est parfaitement réhydratable sans altération dudit tissu et sans rétreint surfacique. Le polyéthylène glycol agit comme un masque dans la structure tridimensionnelle du tissu, qui peut ainsi être stocké à température ambiante 20°C (+/-2°C), dans un conditionnement propre à l'abri de la poussière. De plus, le polyéthylène glycol se rince facilement, il est non toxique pour l'environnement et pour l'opérateur et il est biocompatible. Enfin, le polyéthylène glycol ne gêne en rien la pénétration de l'élastomère en dispersion dans le tissu biologique, lors de la réalisation du collage.

On remarquera que, dans le procédé du document EP 1 785 154, on prévoit que, pour améliorer encore plus la préservation de sa structure tissulaire pendant la lyophilisation, le tissu biologique animal est préalablement traité pendant plusieurs jours par du polyéthylène glycol. On notera cependant qu'un tel traitement, effectué pendant une longue période et donc avec une solution de polyéthylène glycol à faible concentration (de l'ordre de 6% en poids de polyéthylène glycol pour 94% en poids d'eau) n'a pour objet que d'aider à la préservation de la structure du tissu biologique animal lors de la lyophilisation et ne concerne pas la déshydratation de celui-ci, déshydratation qui est obtenue totalement par la lyophilisation.

On remarquera de plus que l'article de RAMSHAW J.A.M. et al. « Précipitation of collagens by polyethylene glycols » , Analytical Biochemistry, Academic Press Inc, New York, vol. 141, n°2, 1er septembre 1984, pages 361-365, XP000600477 concerne :
● le collagène et non pas un tissu animal ;
● la précipitation du collagène en milieu liquide et non pas un collage sur un support, et
● la précipitation du collagène par du polyéthylène glycol et non pas la déshydratation d'un tissu biologique animal par du polyéthylène glycol.
En aucun cas, cet article ne décrit, ni ne suggère de remplacer, avec les mêmes résultats, la déshydration d'un tissu biologique animal par lyophilisation par une déshydration dudit tissu biologique animal par immersion dans un bain constitué d'une solution de polyéthylène glycol à au moins 80% en poids.

Pour l'obtention du matériau hémocompatible composite conformément à la présente invention, on réalise les étapes de base suivantes :
**1**. on commence par fixer chimiquement, de façon connue, le tissu biologique animal, de préférence constitué de péricarde, par tout produit approprié tel qu'un aldéhyde. Dans ce dernier cas, on utilise de préférence le glutaraldéhyde, par exemple à la concentration de 0,625%. Une telle fixation chimique assure au tissu biologique, non antigénicité, stabilité chimique, biologique et physique, et notamment résistance aux variations de température et de contraintes mécaniques ;
**2**. ensuite, le tissu biologique animal est déshydraté par voie chimique par immersion dans un bain constitué d'une solution de polyéthylène glycol à au moins 80% en poids. Ledit bain est avantageusement une solution aqueuse comportant au moins 90% en poids de polyéthylène glycol ou une solution aqueuse comportant au moins 80% en poids de polyéthylène glycol et 10% en poids d'alcool. De plus, le polyéthylène glycol utilisé pour former ledit bain (formule HO-CH₂-(CH₂-O-CH₂)ₙ-CH₂-OH) présente avantageusement une masse molaire comprise entre 100 et 800.
   La durée de l'immersion dudit tissu biologique animal dans ledit bain est de l'ordre de 24 heures et, pendant cette immersion, il est avantageux que ledit bain soit soumis à une légère agitation et que sa température soit au moins égale à la température ambiante (par exemple de l'ordre de 37°C).
   A la fin de l'immersion, ledit tissu biologique animal est sorti dudit bain et l'excédent de la solution de polyéthylène glycol imprégnant ledit tissu biologique est étanché.
**3**. par ailleurs, sur ledit substrat synthétique souple, qui est avantageusement en un élastomère de polyuréthane ou de silicone, on dépose une couche d'une dispersion de la matière constitutive dudit substrat dans un solvant. Par exemple, si ledit substrat est un élastomère de polyuréthane, ladite dispersion contient du polyuréthane biocompatible dans un solvant dépendant du polyuréthane, qui peut être du diméthylacétamide. Cette dispersion, qui peut être déposée sur ledit substrat de toute manière connue (enduction, pulvérisation, etc....) a pour objet de servir d'agent d'adhésion hémocompatible avec le tissu biologique. Puis, sur ladite couche d'agent d'adhésion hémocompatible, on applique ledit tissu biologique déshydraté - qui s'imprègne de ladite dispersion - pour assurer l'adhésion mécanique dudit tissu biologique sur ledit substrat et obtenir ledit matériau composite ;
**4**. après quoi, on procède à l'élimination du solvant dudit agent d'adhésion hémocompatible, par exemple par séchage à chaud, séchage à chaud sous vide et/ou par extraction à chaud dans du sérum physiologique. De préférence, l'élimination du solvant est obtenue par une extraction lente à chaud (par exemple de l'ordre de 40°C), suivie d'une extraction sous vide et complétée par une extraction dans du sérum physiologique.
**5**. enfin, on réhydrate le matériau composite avec du sérum physiologique.
   En plus des étapes de base 1 à 5 décrites ci-dessus, le procédé selon l'invention peut comporter, après l'étape 2 d'immersion du tissu biologique animal dans ledit bain de polyéthylène glycol et avant l'étape 3 d'adhésion dudit tissu biologique animal sur ledit substrat synthétique souple, une ou les deux étapes supplémentaires suivantes :
**6**. séchage dudit tissu biologique animal imbibé de polyéthylène glycol sous atmosphère contrôlée pendant plusieurs heures (par exemple 24 heures) et à une température au moins égale à la température ambiante (par exemple 37°C) ;
**7**. application sur la face dudit tissu biologique animal devant être collée sur ledit substrat synthétique d'un solvant volatil de dégraissage et d'assèchement, tel que l'acétone ou l'éther.

Par ailleurs, l'étape 5 de réhydratation du matériau composite peut être réalisée soit immédiatement, soit de façon différée, après l'étape **4** d'élimination du solvant. Si cette réhydratation est différée après l'étape **4**, ledit matériau composite peut :
- être conservé à l'état déshydraté jusqu'à son utilisation et être réhydraté juste avant celle-ci ; ou
- être conservé dans une solution de polyéthylène glycol, semblable à celle de l'étape **2** de déshydratation, jusqu'à son utilisation, la réhydratation de l'étape **5** étant réalisée juste avant ladite utilisation.

Quel que soit le mode de conservation mentionné ci-dessus, ladite conservation peut être améliorée par une étape de stérilisation, par exemple à l'oxyde d'éthylène ou par rayonnement Y/β.

## Revendications

1. Procédé pour la réalisation d'un matériau hémocompatible comportant un substrat synthétique, résistant et étanche, et un tissu biologique animal, fixé chimiquement afin d'éviter des réactions immunologiques, procédé selon lequel on déshydrate ledit tissu biologique animal, on colle ledit tissu biologique animal déshydraté sur ledit substrat synthétique au moyen d'une dispersion de la matière constitutive dudit substrat synthétique dans un solvant de manière que ladite matière constitutive imprègne ledit tissu biologique animal, puis on élimine ledit solvant,
**caractérisé en ce que** la déshydratation dudit tissu biologique animal est obtenue, sans lyophilisation, uniquement par voie chimique par immersion dudit tissu biologique animal dans un bain constitué d'une solution de polyéthylène glycol à au moins 80% en poids, la durée de l'immersion dudit tissu biologique animal dans ledit bain étant de l'ordre de 24 heures.

2. Procédé selon la revendication 1,
**caractérisé en ce que** ledit bain est une solution aqueuse comportant au moins 90% en poids de polyéthylène glycol.

3. Procédé selon la revendication 1,
**caractérisé en ce que** ledit bain est une solution aqueuse comportant au moins 80% en poids de polyéthylène glycol et 10% en poids d'alcool.

4. Procédé selon l'une quelconque des revendications 1 à 3, **caractérisé en ce que** le polyéthylène glycol de ladite solution présente une masse molaire comprise entre 100 et 800.

5. Procédé selon l'une des revendications 1 à 4,
**caractérisé en ce que** ledit bain présente une température au moins égale à la température ambiante et est agité pendant l'immersion dudit tissu biologique animal.

6. Procédé selon l'une des revendications 1 à 5,
**caractérisé en ce que** l'excédent de la solution de polyéthylène glycol imprégnant ledit tissu biologique animal après immersion dans ledit bain est étanché avant le collage dudit tissu biologique animal sur ledit substrat synthétique.

7. Procédé selon l'une des revendications 1 à 6,
**caractérisé en ce que**, après immersion dans ledit bain et avant collage sur ledit substrat synthétique, ledit tissu biologique animal est séché pendant plusieurs heures à une température au moins égale à la température ambiante.

8. Procédé selon l'une des revendications 1 à 7,
**caractérisé en ce que**, après immersion dans ledit bain et avant collage sur ledit substrat synthétique, on applique sur la face dudit tissu biologique animal dirigée vers ledit substrat synthétique un solvant volatil de dégraissage et d'assèchement.

9. Procédé selon la revendication 8,
**caractérisé en ce que** ledit solvant de dégraissage et d'assèchement est choisi entre l'acétone et l'éther.

10. Procédé selon l'une quelconque des revendications 1 à 9,
**caractérisé en ce qu'**on réhydrate ledit matériau hémocompatible immédiatement après l'élimination dudit solvant de la dispersion de la matière constitutive du substrat synthétique.

11. Procédé selon l'une quelconque des revendications 1 à 9,
**caractérisé en ce qu'**on réhydrate ledit matériau hémocompatible de façon différée après l'élimination dudit solvant de la dispersion de la matière constitutive du substrat synthétique.

12. Procédé selon la revendication 11,
**caractérisé en ce que**, après l'élimination dudit solvant de la dispersion de la matière constitutive du substrat synthétique, on conserve ledit matériau hémocompatible à l'état déshydraté et on le réhydrate juste avant son utilisation.

13. Procédé selon la revendication 11,
**caractérisé en ce que**, après l'élimination dudit solvant de la dispersion de la matière constitutive du substrat synthétique, on conserve ledit matériau hémocompatible dans une solution de polyéthylène glycol et on le réhydrate juste avant son utilisation.

## Claims

1. Method for producing a hemocompatible material comprising a synthetic substrate, which is resistant and impermeable, and an animal biological tissue, which is chemically fixed in order to prevent immunological reactions, according to which method said animal biological tissue is dehydrated, said dehydrated animal biological tissue is adhered to said synthetic substrate by means of a dispersion of the constituent substance of said synthetic substrate in a solvent in such a way that said constituent substance impregnates said animal biological tissue, and then said solvent is eliminated,
**characterised in that** said animal biological tissue is dehydrated solely chemically, without freeze drying, by immersing said animal biological tissue in a bath consisting of a solution comprising at least 80 % by weight polyethylene glycol, said animal biological tissue being immersed in said bath for approximately 24 hours.

2. Method according to claim 1, **characterised in that** said bath is an aqueous solution comprising at least 90 % by weight polyethylene glycol.

3. Method according to claim 1, **characterised in that** said bath is an aqueous solution comprising at least 80 % by weight polyethylene glycol and 10 % by weight alcohol.

4. Method according to any of claims 1 to 3, **characterised in that** the polyethylene glycol of said solution has a molar mass of between 100 and 800.

5. Method according to any of claims 1 to 4, **characterised in that** said bath has a temperature at least equal to the ambient temperature and is stirred during immersion of said animal biological tissue.

6. Method according to any of claims 1 to 5, **characterised in that** the excess of the polyethylene glycol solution impregnating said animal biological tissue after immersion in said bath is sealed before said animal biological tissue is adhered to said synthetic substrate.

7. Method according to any of claims 1 to 6, **characterised in that**, after immersion in said bath and before adhesion to said synthetic substrate, said animal biological tissue is dried for several hours at a temperature at least equal to the ambient temperature.

8. Method according to any of claims 1 to 7, **characterised in that**, after immersion in said bath and before adhesion to said synthetic substrate, a volatile degreasing and drying solvent is applied to the face of said animal biological tissue facing said synthetic substrate.

9. Method according to claim 8, **characterised in that** said degreasing and drying solvent is selected from acetone and ether.

10. Method according to any of claims 1 to 9, **characterised in that** said hemocompatible material is rehydrated immediately after said solvent is eliminated from the dispersion of the constituent substance of the synthetic substrate.

11. Method according to any of claims 1 to 9, **characterised in that** said hemocompatible material is rehydrated at a later point in time after said solvent is eliminated from the dispersion of the constituent substance of the synthetic substrate.

12. Method according to claim 11, **characterised in that**, after said solvent is eliminated from the dispersion of the constituent substance of the synthetic substrate, said hemocompatible material is kept in a dehydrated state and is rehydrated just before it is used.

13. Method according to claim 11, **characterised in that**, after said solvent is eliminated from the dispersion of the constituent substance of the synthetic substrate, said hemocompatible material is kept in a polyethylene glycol solution and is rehydrated just before it is used.

## Patentansprüche

1. Verfahren zur Herstellung eines blutverträglichen Materials, umfassend ein widerstandsfähiges und dichtes synthetisches Substrat und ein tierisches biologisches Gewebe, das chemisch fixiert ist, um immunologische Reaktionen zu vermeiden, wobei bei diesem Verfahren das tierische biologische Gewebe dehydriert wird, das dehydrierte tierische biologische Gewebe mittels einer Dispersion des Stoffes, welches das synthetische Substrat bildet, in einem Lösemittel an das synthetische Substrat geklebt wird, derart, dass das bildende Stoff das tierische biologische Gewebe imprägniert, und dann das Lösemittel entfernt wird, **dadurch gekennzeichnet, dass** die Dehydrierung des tierischen biologischen Gewebes, ohne Gefriertrocknung, einzig auf chemischem Wege erhalten wird durch Eintauchen des tierischen biologischen Gewebes in ein Bad, das von einer Lösung mit mindestens 80 Gew.-% Polyethylenglykol gebildet ist, wobei die Eintauchdauer des tierischen biologischen Gewebes in das Bad in der Größenordnung von 24 Stunden liegt.

2. Verfahren nach Anspruch 1, **dadurch gekennzeichnet, dass** das Bad eine wässrige Lösung mit mindestens 90 Gew.-% Polyethylenglykol ist.

3. Verfahren nach Anspruch 1, **dadurch gekennzeichnet, dass** das Bad eine wässrige Lösung mit mindestens 80 Gew.-% Polyethylenglykol und 10 Gew.-% Alkohol ist.

4. Verfahren nach einem der Ansprüche 1 bis 3, **dadurch gekennzeichnet, dass** das Polyethylenglykol der Lösung eine Molmasse zwischen 100 und 800 aufweist.

5. Verfahren nach einem der Ansprüche 1 bis 4, **dadurch gekennzeichnet, dass** das Bad eine Temperatur aufweist, die mindestens gleich der Umgebungstemperatur ist, und während des Eintauchens des tierischen biologischen Gewebes gerührt wird.

6. Verfahren nach einem der Ansprüche 1 bis 5, **dadurch gekennzeichnet, dass** der Überschuss an Polyethylenglykol-Lösung, die nach dem Eintauchen in das Bad das tierische biologische Gewebe imprägniert, vor dem Kleben des tierischen biologischen Gewebes an das synthetische Substrat abgedichtet wird.

7. Verfahren nach einem der Ansprüche 1 bis 6, **dadurch gekennzeichnet, dass**, nach dem Eintauchen in das Bad und vor dem Kleben an das synthetische Substrat, das tierische biologische Gewebe über mehrere Stunden bei einer Temperatur getrocknet wird, die mindestens gleich der Umgebungstemperatur ist.

8. Verfahren nach einem der Ansprüche 1 bis 7, **dadurch gekennzeichnet, dass**, nach dem Eintauchen in das Bad und vor dem Kleben an das synthetische Substrat, ein flüchtiges Lösemittel zur Entfettung und Entwässerung auf die Fläche des tierischen biologischen Gewebes aufgetragen wird, die in Richtung des synthetischen Substrats gerichtet ist.

9. Verfahren nach Anspruch 8, **dadurch gekennzeichnet, dass** das Lösemittel zur Entfettung und Entwässerung aus Aceton und Ether gewählt ist.

10. Verfahren nach einem der Ansprüche 1 bis 9, **dadurch gekennzeichnet, dass** das blutverträgliche Material sofort nach dem Entfernen des Lösemittels aus der Dispersion des Stoffes, welches das synthetische Substrat bildet, rehydriert wird.

11. Verfahren nach einem der Ansprüche 1 bis 9, **dadurch gekennzeichnet, dass** das blutverträgliche Material verzögert nach dem Entfernen des Lösemittels aus der Dispersion des Stoffes, welches das synthetische Substrat bildet, rehydriert wird.

12. Verfahren nach Anspruch 11, **dadurch gekennzeichnet, dass**, nach dem Entfernen des Lösemittels aus der Dispersion des Stoffes, welches das synthetische Substrat bildet, das blutverträgliche Material im dehydrierten Zustand aufbewahrt und kurz vor seiner Verwendung rehydriert wird.

13. Verfahren nach Anspruch 11, **dadurch gekennzeichnet, dass**, nach dem Entfernen des Lösemittels aus der Dispersion des Stoffes, welches das synthetische Substrat bildet, das blutverträgliche Material in einer Polyethylenglykol-Lösung aufbewahrt und kurz vor seiner Verwendung rehydriert wird.
